(19) **Europäisches Patentamt**
**European Patent Office**
**Office européen des brevets**

(11) **EP 3 185 069 A1**

(12) **EUROPEAN PATENT APPLICATION**

(43) Date of publication:
**28.06.2017 Bulletin 2017/26**

(51) Int Cl.:
***G02F 1/1335*** *(2006.01)* *G02B 27/00* *(2006.01)*

(21) Application number: **15202241.4**

(22) Date of filing: **22.12.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **IMEC VZW
3001 Leuven (BE)**

(72) Inventors:
• **GENOE, Jan
3001 Leuven (BE)**
• **HEREMANS, Paul
3001 Leuven (BE)**

(74) Representative: **DenK iP
Hundelgemsesteenweg 1116
9820 Merelbeke (BE)**

(54) **VISION CORRECTION DISPLAY**

(57) A display (100) for generating an image with a perceived depth (104) of image pixels differing from the actual depth (103) of the image pixels (300), the display comprising: an array of microlenses (101) having a first pitch along a first direction (X); a plurality of arrays of light sources (102) underneath the array of microlenses, an array of light sources having a second pitch along the first direction, the second pitch differing from the first pitch; and means for addressing the plurality of arrays of light sources. Each array of light sources corresponds to one perceived image pixel, and each light source in an array is present underneath a different microlens. The means for addressing the arrays of light sources is adapted for addressing each light source in one array of light sources in unison.

**FIG. 2**

**Description**

**Technical field of the invention**

The present invention relates to electronic displays and more specifically to electronic displays which provide a level of vision correction.

**Background of the invention**

In our society, an increasingly large number of people suffer from deteriorated eye sight and are thus in need of some form of vision correction. In particular, a large number of people with an age beyond 50 need reading glasses. Without glasses, they have difficulties to read texts at distances below 50 cm. At the same time, people are exposed to and interact with a growing amount of devices that make use of some form of electronic display, such as watches, phones, smart wearables, etc. Thus, the ability to see and read these displays, which are generally located at a relatively close distance (< 50 cm), becomes more and more relevant.

Corrective eye wear, such as glasses and contact lenses, have historically been used to address this issue. More recently, corrections through surgery have also become possible. Yet, these solutions are not without their own problems. Indeed, while a person with myopia (near-sightedness) might be accustomed to wearing glasses or lenses throughout their day to day activities, a person with hyperopia (far-sightedness) or presbyopia might not always have their reading glasses readily available. This is in particular a problem when displays need to be read during activities in which wearing corrective eye wear is impractical or even unsafe, e.g. reading out a heart rate monitor during sports or looking at a dashboard display while driving. Surgery, on the other hand, is very intrusive and can potentially be dangerous.

Specifically with respect to electronic displays, a number of solutions have been proposed that aim at modifying the displayed signal so that a particular user perceives a corrected image, without the need for any corrective eye wear. These approaches include changing the displayed signal based on light-field computations (F.C. Huang et al., ACM Trans. Graph., 2012, 31.6: 185), making use of so called glasses-free 3D displays to create a multi-depth image (V. Pamplona et al, ACM Trans. Graph., 2012, 31.4: 81) and a combination of both (F.C. Huang et al., ACM Trans. Graph., 2014, 33.4: 59). These approaches have in common that they can, in principle, produce a corrected image that is tailored to the individual requirements of the user and that they can address even higher order aberrations, which are currently not correctable by corrective eye wear. Nevertheless, each of these approaches has considerable downsides which prevent them from being implemented in current devices, including, respectively: huge loss of contrast, large loss of resolution and computational requirements which are not currently always feasible in portable devices.

As such, there exists a need for electronic displays providing vision correction for users, implementable in devices within the current state-of-the-art.

**Summary of the invention**

It is an object of the present invention to provide good apparatus and methods for read-out of electronic displays while providing vision correction.

The above objective is accomplished by a method and device according to embodiments of the present invention.

It is an advantage of embodiments of the present invention that a vision corrected image may be obtained without loss in contrast, resolution or the need for additional computational requirements.

It is an advantage of embodiments of the present invention that a vision corrected image may be obtained without a significant increase in the footprint of the electronics.

It is an advantage of embodiments of the present invention that a vision correction display may be obtained with no or minimal changes in the driving electronics of the display.

It is an advantage of embodiments of the present invention that vision correction displays suitable for myopia, hyperopia and presbyopia may be obtained, depending on the design choices of the display.

It is an advantage of embodiments of the present invention that a display with both a normal and a vision correction mode of operation may be created.

In a first aspect, the present invention relates to a display for generating an image with a perceived depth of image pixels differing from the actual depth of the image pixels. The display comprises:

> an array of microlenses having a first pitch along a first direction;
> a plurality of arrays of light sources underneath the array of microlenses, an array of light sources having a second pitch along the first direction, the second pitch differing from the first pitch; and
> means for addressing the plurality of arrays of light sources.

Each array of light sources corresponds to one perceived image pixel and each light source in an array is present underneath a different microlens. The means for addressing the arrays of light sources is adapted for addressing each light source in one array of light sources in unison.

It is an advantage of embodiments of the present invention that an image with a perceived depth of image pixels different from the actual depth of image pixels may be obtained, without loss of resolution.

In embodiments of the present invention, the array of light sources may comprise three or more light sources, preferably 20 or more light sources, yet more preferably 50 or more, most preferably 90 or more. Each light source emits light isotropically. Only after the light has passed through a microlens, the light is collimated to a single angle (or a narrow range of angles). The different angles obtained for the different light sources of an array are the result of the difference in pitch between the light sources and the microlenses, leading to a difference in relative position of a light source w.r.t. the corresponding microlens for the different microlenses. The more light sources are present underneath a single microlens, the more angles light can be sent out at.

In embodiments of the present invention, a row of image pixels may be formed by two or more arrays of light sources.

In embodiments of the present invention, the means for addressing each light source in one array of light sources in unison comprises each light source in the array being hardwired to a single driving transistor. By the hardwiring of light sources in an array to a single transistor, constraints on transistors, in particular with respect to their footprint, are alleviated, at the trade-off of not having selectable perceived depth of image pixels. The perceived depth of image pixels is at a constant value, fixed at design time. It is an advantage of embodiments of the present invention that less transistors are required.

In embodiments of the present invention, the light sources are arranged in a predetermined pattern, such as for instance a staggered and/or shifted pattern. The light sources are electrically contacted by means of contact lines. The pattern is such that the contact lines do not intersect, so as to not short-circuit one another.

An array of light sources may comprise subgroups each comprising a plurality of light sources. The subgroups may be shifted with respect to one another along the first direction and the plurality of light sources within a subgroup may be shifted with respect to another light source of the same subgroup both perpendicular to and along the first direction. Optimal placement of the light sources within the subgroups and within the arrays may be determined taking into account that light sources of an array need to be connected by a hardwired connection to a single transistor, and that such connection lines of different arrays of light sources should not touch one another. Hereto, contact lines that connect two light sources in a subgroup of light sources may make an angle $\alpha$ with the first direction, wherein the angle $\alpha$ is smaller than $\arctan\left(\frac{H}{mP}\right)$, with H the height of the row of image pixels, m the number of light sources in the subgroup and P the size of the second pitch. In embodiments of the present invention, contact lines that connect two light sources in a subgroup of light sources may make an angle $\alpha$ with the first direction, wherein the angle $\alpha$ is larger than $\arccos\left(\frac{H}{nT}\right)$, with H the height of the row of image pixels, n the number of contact lines present in an image pixel and T the minimum contact line pitch.

In embodiments of the present invention, the second pitch between the light sources is smaller than the first pitch between the microlenses in the array. This allows the display to be used for presbyopia.

In particular embodiments of the present invention, the perceived depth of the image pixels differs from the actual depth of the image pixels by 10 to 200 cm, more preferably by 20 to 100 cm, yet more preferably by 25 to 50 cm.

In particular embodiments, the array of microlenses comprises cylindrical microlenses oriented perpendicular to the first direction. These embodiments are easier to implement than embodiments with spherical lenses.

A display according to embodiments of the present invention may comprise organic light emitting diodes as light sources.. These can easily and cheaply be manufactured at high resolution.

In a display according to embodiments of the present invention, one or more additional light sources may furthermore be provided per pixel, such that in an alternative mode of operation an image may be created with a perceived depth equal to the depth of the source. This way, a display with a dual function can be provided: a first function being the "normal" visualisation mode of an image, and a second function being the vision enhanced mode. Switching from one mode to the other one may for example be accomplished by pressing a button.

In a second aspect, the present invention relates to a method for generating an image with a perceived depth of image pixels differing from the actual depth of the image pixels. The method comprises, in an electronic device, for instance an electronic mobile device, comprising a display, the display comprising an array of microlenses having a first pitch along a first direction and a plurality of arrays of light sources underneath the array of microlenses, the arrays of light sources having a second pitch along the first direction differing from the first pitch, addressing each light source in one array of light sources in unison. Addressing a plurality of light sources in unison relaxes the requirements on the number of transistors that need to be present to drive the light sources, hence high resolution images can be obtained.

In a third aspect, the present invention relates to a method for designing a display for generating an image with a perceived depth of image pixels differing from the actual depth of the image pixels. The design method comprises choosing a number x of pixels per row in the display and a number y of rows, wherein the rows are oriented in a first direction; choosing a number n of emission angles for constructing perceived pixels; and choosing a number m of rows of light sources in each physical pixel. Choosing the number x of pixels per row in the display and the number y of rows, generally relates to a choice of the size and pixel density of the screen. Then, once the above design parameters are selected, the method comprises providing an array of microlenses with a first pitch in the first direction X. A plurality of arrays of light sources with a second pitch P in the first direction X differing from the first pitch is provided, wherein each array comprises n light sources and contact lines electrically contacting the n light sources. The light sources are addressable in unison. They are ordered into subgroups of m light sources. The subgroups are shifted with respect to one another in the first direction X. The m light sources in a subgroup are shifted both in the first direction X and perpendicular to the first direction X such that the contact line contacting the m light sources of a subgroup makes an angle α with the first direction X, so that arccos $\left(\frac{H}{nT}\right) < \alpha < \arctan\left(\frac{H}{mP}\right)$, in which H is the height of a row of image pixels and T is the minimum contact line pitch. The plurality of arrays of light sources are arranged, without overlapping them, such that only one light source per array of light sources is present beneath a microlens, while n arrays of light sources are represented in a physical pixel.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

**Brief description of the drawings**

FIG. 1 shows a schematic representation of a single perceived pixel, constructed from 9 physical pixels, with a depth differing from the real depth of the physical pixels, according to embodiments of the present invention.

FIG. 2 shows an array of light sources below an array of microlenses according to embodiments of the present invention.

FIG. 3 and FIG. 4 show angle requirements for contact lines in particular embodiments of the present invention.

FIG. 5 shows the trajectory of different light rays, emitted from a same physical pixel, according to particular embodiments of the present invention.

FIG. 6 shows an OLED strip which may be used as a light source in particular embodiments of the present invention.

FIG. 7 shows a way of arranging a plurality of arrays of light sources over three physical pixels comprising twenty light sources each.

FIG. 8 and FIG. 9 show different connection schemes for operating displays according to particular embodiments of the present invention either in a vision correction or non-vision correction mode.

In the different figures, the same reference signs refer to the same or analogous elements.

**Description of illustrative embodiments**

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

The terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

In a first aspect, the present invention relates to a display 100 for generating an image with a perceived depth 104 of image pixels differing from the actual depth 103 of the image pixels. The display 100 comprises:

> an array of microlenses 101 having a first pitch along a first direction X;
> a plurality of arrays of light sources 102 underneath the array of microlenses 101, an array of light sources 102 having a second pitch along the first direction X, the second pitch differing from the first pitch; and
> means for addressing the plurality of arrays of light sources 102.

Each array of light sources 102 corresponds to one perceived image pixel and each light source 102 in an array is present underneath a different microlens 101. The means for addressing the arrays of light sources 102 is adapted for addressing each light source 102 in one array of light sources 102 in unison.

The perceived depth 104 of image pixels is the apparent depth at which the pixels appear to be located, as perceived by an observer of the image pixels; conversely, the actual depth 103 of the image pixels is the real physical distance between the observer and image pixels.

Furthermore, a distinction needs to be made between a perceived pixel 105 and a physical pixel: a perceived pixel 105 is a basic unit of the image, as it appears to the observer, located at a perceived depth 104; a physical pixel 300 on the other hand is a basic unit of the display 100 and is located at the actual depth 103. In the present invention, a physical pixel 300 typically has a rhomboid shape, and comprises a plurality of light sources 102. It typically has a width substantially equal to, or even equal to, the width of a microlens 101 and typically aligns on two opposing sides with sides of the microlens 101.

The first direction X is typically the horizontal direction, although the present invention is not limited thereto. The difference between the first pitch and the second pitch is such that each light source 102 in an array is present underneath a different microlens 101. Moreover, the difference between the first pitch and the second pitch is typically such that each light source 102 in an array is present underneath subsequent microlenses 101. The second pitch is thus typically between 0.5 and 2 times the first pitch, preferably between 0.75 and 1.5 times, yet more preferably between 0.85 and 1.15 times, most preferably between 0.95 and 1.05 times, and different from 1.

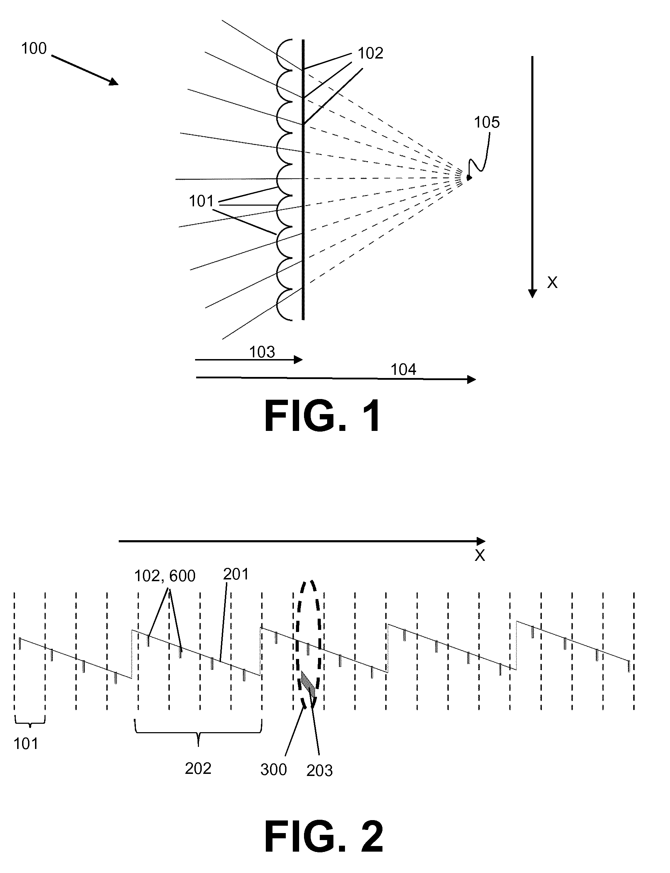

Reference is made to FIG. 1. Upon activating an array of light sources 102, the light coming from each light source 102 in the array, after passing through its corresponding microlens 101, travels under a different angle, thereby mimicking the light that would be emitted by a single pixel 105 at a predetermined perceived depth 104. An array consisting of two or more light sources 102 thus advantageously allows an observer to see a corresponding perceived pixel 105 at a perceived depth 104 differing from the actual depth 103 of the two or more light sources 102.

Addressing each light source 102 in an array of light sources 102 in unison advantageously leads to a considerable simplification of the addressing means, resulting in a significant reduction in the footprint of the addressing means.

In embodiments of the present invention, an array of light sources 102 may comprise three or more light sources, more preferably twenty or more, yet more preferably fifty or more, most preferably ninety or more.

While two angles of light emission, corresponding to two light sources 102, can in principle be sufficient to create a differing depth perception, it also severely limits the viewing angle under which this depth perception is correctly observed. Furthermore, while the human brain is able to compile this emission under two angles into a perceived pixel 105, the discrepancy with an actual light source located at the perceived distance, which emits light under an infinite number of angles, leads to headaches in some users, especially over prolonged viewing. As such, increasing the number of light sources 102, thereby increasing the number of emission angles, advantageously approaches a more realistic mimicking of the perceived pixel 105, which leads to larger viewing angles and a more pleasant viewing experience. It is not expected that a human observer is able to perceive a difference in the amount of emission angles, i.e. light sources 102 in an array, when the number of light sources in an array is increased beyond 180. An array of light sources 120 may thus, for example, comprise up to 180 light sources 120.

In embodiments of the present invention, a row of image pixels may be formed by two or more arrays of light sources 102.

Since one array of light sources 102 corresponds to one perceived pixel 105, a row of pixels may advantageously be formed by using a number of arrays of light sources corresponding to the number of pixels in the row. By extension, a 2D image may be obtained by using multiple rows of pixels.

In preferred embodiments, at least two arrays of light sources 102 are partially present under a single microlens 101.

By a proper arrangement of the arrays, such that their light sources 102 do not overlap one another, multiple light sources 102, each corresponding to different arrays, may be present under a single microlens 101. This advantageously allows perceived pixels 105 to be created which are sufficiently close to one another so that a continuous image, without gaps, may be observed.

In embodiments of the present invention, the means for addressing each light source 102 in one array of light sources 102 in unison may comprise each light source 102 in the array being hardwired to a single driving transistor.

Addressing each light source 102 in an array by means of a single driving transistor advantageously reduces the number for driving transistors needed and thus significantly reduces the footprint of the driving electronics.

In embodiments of the present invention, the light sources 102 may be arranged in a predetermined pattern and may be electrically contacted by means of contact lines 201; the pattern being such that the contact lines 201 do not intersect.

The light sources 102 can be arranged in such a way that the contact lines 201 that contact them do not unduly overlap; advantageously allowing the arrays of light sources to be addressed separately. This is particularly relevant when multiple light sources 102, corresponding to multiple arrays, are present beneath a single microlens 101.

In embodiments of the present invention, the array of light sources 102 may comprise subgroups 202 each comprising a plurality of light sources 102; the subgroups 202 being shifted with respect to one another along the first direction X and the plurality of light sources 102 within a subgroup 202 being shifted with respect to another light source 102 of the same subgroup 202 both perpendicular to and along the first direction X.

With reference to FIG. 2, a good pattern for arranging the light sources 102 may advantageously be achieved by arranging the light sources 102 within an array of light sources along a path consisting of a succession of slanted lines.

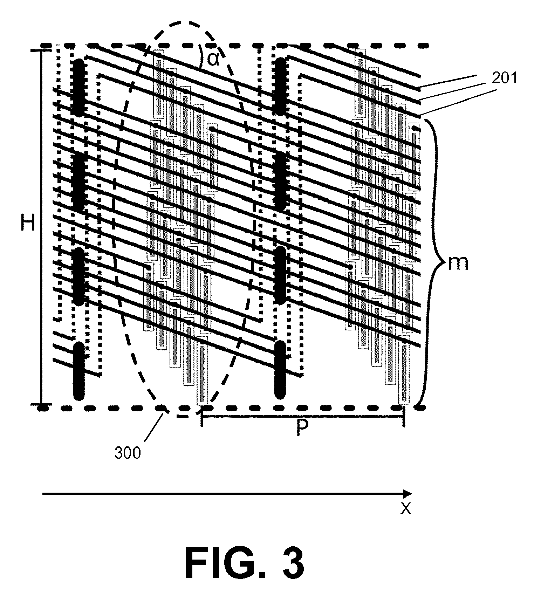

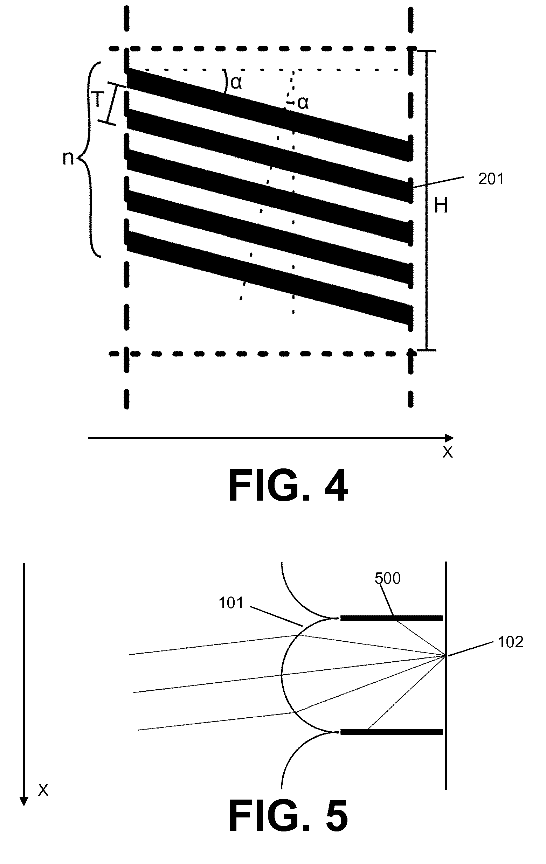

In embodiments of the present invention, contact lines 201 that connect two light sources 102 in a subgroup 202 of light sources 102 may make an angle $\alpha$ with the first direction X. Light sources need to be placed within the height H of a pixel 300. The angle $\alpha$ is smaller than $\arctan\left(\dfrac{H}{mP}\right)$, wherein H is the height of the row of image pixels, m is the number of light sources in the subgroup 202 (four in the example illustrated in FIG. 3) and P is the size of the second pitch. The angle $\alpha$ is preferably larger than $\arccos\left(\dfrac{H}{nT}\right)$, wherein H is the height of the row of image pixels, n is the number of contact lines 201 present in an image pixel (twenty in the example illustrated in FIG. 3 and five in the example illustrated in FIG. 4) and T is the minimum contact line pitch.

Referring to FIG. 4, since the contact lines 201 have a finite width and since the width and placement of these contact lines 201 during manufacturing cannot be perfectly controlled, the angle $\alpha$ of the contact lines 201 is advantageously larger than $\arccos\left(\dfrac{H}{nT}\right)$; otherwise there is a risk of shorting or overlap between two different contact lines 201. The minimum contact line pitch T is defined here as the sum of the contact line width and the minimum separation distance between two contact lines 201. This minimum separation distance is the distance needed to prevent shorting or overlap between contact lines 201 due to slight variations in contact line width and/or contact line placement during manufacturing. In general, the minimum contact line pitch T depends on the manufacturing technology that is used for fabricating the light sources and contact lines.

In embodiments, the second pitch may be smaller than the first pitch.

When the second pitch is smaller than the first pitch, the perceived depth 104 of the image pixels is larger than the actual depth 103; thereby creating an image that is perceived to be further away, advantageously allowing people with presbyopia and/or hyperopia to more correctly observe the image.

In other embodiments, the second pitch may be larger than the first pitch.

When the second pitch is larger than the first pitch the perceived depth 104 of the image pixels is smaller than the actual depth 103; thereby creating an image that is perceived to be closer, advantageously allowing people with myopia to more correctly observe the image.

In embodiments, the perceived depth 104 of the image pixels may differ from the actual depth of the image pixels by 10 to 200 cm, more preferably by 20 to 100 cm, yet more preferably by 25 to 50 cm.

For most users with presbyopia, hyperopia or myopia, a difference in depth of the perceived image, compared to the actual depth of the image on the screen, in the order of a few decimetres advantageously results in a significant improvement in the observed image, for instance in readability thereof.

In embodiments of the present invention, the array of microlenses 101 may comprise cylindrical microlenses oriented perpendicular to the first direction X.

In general, the impression of depth is distinguished by a human observer through the horizontal (direction X) angular difference seen by both eyes in an effect called binocular disparity. Moreover, when additional depth information is required, the human observer will generally instinctively attempt to gain this information by a horizontal displacement, i.e. moving and/or turning his head and/or body horizontally. As such, for most embodiments, the angular perception in the horizontal direction provided by cylindrical microlenses oriented perpendicular thereto, as opposed to e.g. spherical microlenses, is advantageously sufficient to create a depth perception.

In embodiments, the array of light sources 102 may comprise organic light emitting diodes. Organic light emitting diodes can advantageously be made very small, in the order of a few micrometres, allowing a multitude of individual light sources to fit within a normal pixel size.

In embodiments, the light sources 102 may be confined so that they can each only emit light through the microlens 101 that directly overlays them.

In preferred embodiments, the light sources 102 may be so confined through means of an absorbing material, which absorbs light that would emit through a different microlens. In the illustration in FIG. 5, each light source 102 is aligned with the microlens 101 directly overlaying it to emit light under the specific angle that is required for that light source. However, this is not the case for other microlenses 101. As such, the light sources 102 advantageously only emit light through the microlens 101 that directly overlays them, avoiding cross-talk. This may advantageously be achieved by providing an absorbing material 500 between different pixels. The absorbing material 500 absorbs light that would be emitted by a light source 102 provided underneath a different microlens 101 (hence in a different pixel), so as to prevent that light from being received by the first microlens 101.

In embodiments of the present invention, the display 100 may furthermore comprise one or more additional light sources 203 per pixel 300 such that in an alternative mode of operation an image may be created with a perceived depth equal to the depth of the source.

A screen which may be operated in both a vision correction and a normal, non-vision correction, mode advantageously allows the screen to be used both by people who require the vision correction and those who do not. In embodiments of the present invention, switching the mode of operation may be achieved by a software implementation. In other embodiments, switching the mode of operation may be achieved by a hardware implementation. For example, the device comprising the screen may comprise a vision correction button or switch which triggers a change in the mode of operation.

In a second aspect, the present invention relates to a method for generating an image with a perceived depth of image pixels differing from the actual depth of the image pixels. The method comprises, in an electronic device, for instance an electronic mobile device, comprising a display, the display comprising an array of microlenses 101 having a first pitch along a first direction and a plurality of arrays of light sources 102 underneath the array of microlenses 101, the arrays of light sources 102 having a second pitch along the first direction differing from the first pitch, addressing each light source 102 in one array of light sources 102 in unison.

In a third aspect, the present invention relates to a method for designing a display for generating an image with a perceived depth 104 of image pixels differing from the actual depth 103 of the image pixels. The design method comprises choosing a number x of pixels per row in the display and a number $y$ of rows, wherein the rows are oriented in a first direction; choosing a number n of emission angles for constructing perceived pixels; and choosing a number m of rows of light sources in each physical pixel. Choosing the number x of pixels per row in the display and the number $y$ of rows, generally relates to a choice of the size and pixel density of the screen.

Then, once the above design parameters are selected, the method comprises providing an array of microlenses 101 with a first pitch in the first direction X. A plurality of arrays of light sources 102 with a second pitch P in the first direction X differing from the first pitch is provided, wherein each array comprises n light sources 102 and contact lines 201 electrically contacting the n light sources 102. The light sources are addressable in unison. They are ordered into subgroups 202 of m light sources 102. The subgroups 202 are shifted with respect to one another in the first direction X. The m light sources 102 in a subgroup 202 are shifted both in the first direction X and perpendicular to the first direction X such that the contact line 201 contacting the m light sources 102 of a subgroup 202 makes an angle $\alpha$ with the first direction X, so that $\arccos\left(\frac{H}{nT}\right) < \alpha < \arctan\left(\frac{H}{mP}\right)$, in which H is the height of a row of image pixels and T is the minimum contact line pitch. The plurality of arrays of light sources 102 are arranged, without overlapping them, such that only one light source 102 per array of light sources 102 is present beneath a microlens 101, while n arrays of light sources 102 are represented in a physical pixel.

A higher number n of emission angles for constructing perceived pixels typically leads to an increased viewing angle for the perceived pixels and a more pleasant viewing experience for the observer.

Choosing the number *m* of rows of light sources 102 in each physical pixel typically relates to bridging a non-emissive gap between two adjacent light sources 102 in a row by staggering a plurality of rows of light sources 102. For example, a 15 μm wide light source 102 may have an emissive area of 5 μm and a minimum separation distance with an adjacent light source of 5 μm, such that there is a gap of 15 μm between the emissive areas of 2 adjacent light sources in a row. In this case, the gap may be completely bridged by staggering an additional 3 rows of light sources 102 above or below the first row, each shifted by 5 μm with respect to the previous row.

Arranging the plurality of arrays of light sources 102 without overlapping them, entails arranging them in such a way that a first array of light sources 102 does not directly make electrical contact to a second array of light sources 102, through the light sources 102 and/or contact lines 201 that the arrays are comprised of.

The invention will now be further clarified by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

Example 1: vision correction display with dual mode of operation, suitable for normal vision and presbyopia/hyperopia

In order to build up the display, a multitude of OLED stripes are used as the light sources 102, located below an array of cylindrical microlenses 101 with a focal length $f_L$ of 500 μm and a pitch L of 300 μm.

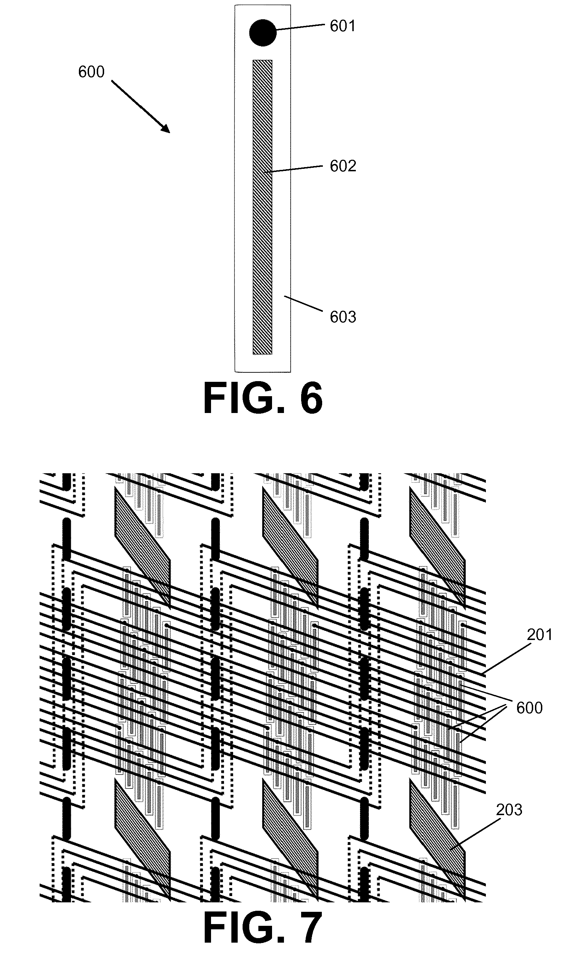

Reference is made to FIG. 6. The OLED stripes 600 comprise a top electrode 601 and an electroluminescent area 602, for example measuring 90 μm by 5 μm, on top of a bottom electrode 603.

With reference to FIG. 2, the multitude of OLED stripes 600 are divided into arrays of OLED stripes, the arrays oriented substantially perpendicular to the cylindrical microlenses 101. For instance, the cylindrical microlenses may be placed vertically, covering a column of image pixels, while the arrays of OLED stripes may be oriented substantially horizontally, covering a row of image pixels. As indicated in FIG. 2, an array of OLED stripes may comprise a plurality of subgroups 202 of such OLED stripes, wherein OLEDs in a subgroup 202 are located on a slanted line, under an angle with respect to a horizontal line, while the combination of all subgroups 202 of the array has a substantially horizontal direction. The directions vertical and horizontal as set out here are only for fixing the mind, and are not necessarily referring to actual physical orientations or directions. This means that, in embodiments of the present invention, columns of image pixels could be placed horizontally or under a different angle, and rows of pixels could be placed vertically or under a different angle.

Each array consists of 20 OLED stripes, with a pitch P of 295 μm, hardwired together to a single driving transistor. Only one array is illustrated in FIG. 2. Each of the 20 OLED stripes is located underneath a single cylindrical microlens and, together, the array forms a single perceived pixel 105 with a perceived depth 104.

Based on Gabor's theory on superlenses (1941) and given the values for the focal length $f_L$ of the microlenses, their pitch L and the pitch of an array of OLED stripes P, the perceived depth change F can be calculated from the following formula:

$$F = f_L \left( 1 + \frac{L}{L - P} \right)$$

$$\Leftrightarrow F = 500 \left( 1 + \frac{300}{300 - 295} \right) \mu m = 30.5 \text{ cm}$$

With the above parameters for the arrays of microlenses and OLED stripes, a perceived image pixel is thus created with an additional depth of 30.5 cm compared to the depth of the physical pixels.

An additional OLED 203, with an area sufficiently similar to the combined area of the 20 OLED stripes 600, is located below, and towards the middle of, the array of OLED stripes. This additional light source 203 is used when the display is operating in its normal, non-vision correction, mode.

This is illustrated in FIG. 7. The display 100 comprises a plurality of arrays of OLED stripes 600: one for each perceived pixel in the display. Equally, there may be one additional OLED 203, i.e. the normal mode light source, for each pixel in the display. As such, each physical pixel of the display 100 comprises a portion of a single cylindrical microlens 101, 20 OLED stripes corresponding to 20 different OLED arrays and one additional non-vision correction OLED 203.

In order to avoid undue electrical contact between the different arrays of OLED stripes 600, the arrays are displaced slightly with respect to one another (see FIG. 7) and the OLED stripes 600 within an array are arranged in a predetermined pattern (see FIG. 2). The pattern consists of subgroups 202 comprising OLED stripes 600 arranged next to one another along the length (X direction) of the array, while subsequent OLED stripes 600 within a subgroup 202 are shifted both along and perpendicular to the length of the array; in other words, the OLED stripes are placed along a succession of slanted lines. Contact lines 201 connecting the OLED stripes 600 within a subgroup 202 are located in a level above the OLED stripes 600 and run diagonally across the subgroup 202. Contact lines 201 connecting different subgroups 202, on the other hand, run perpendicular to the length (X direction) of the array and are located in the same plane as the OLED top electrodes 601.

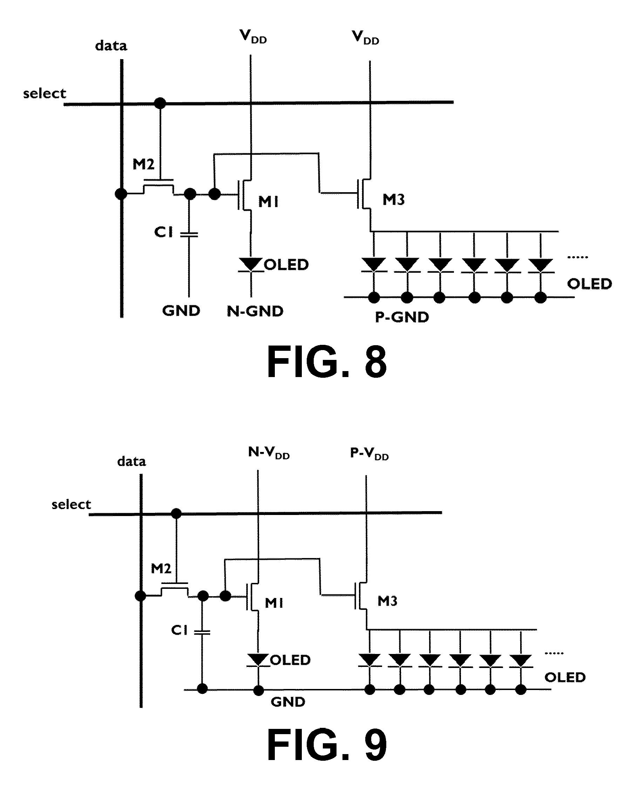

FIG. 8 shows an example of a transistor configuration that may be used for switching between a normal vision mode and a corrected vision mode. The approach used in the example shown uses two ground lines: a presbyopia mode ground line (P-GND) and a normal ground line (N-GND). FIG. 8 illustrates that, by selecting the appropriate ground line, either a normal or vision corrected pixel can be activated, and, by extension, the display 100 can be operated in either a normal or a vision corrected mode. Beyond this selection mechanism, the driving electronics can be the same as in traditional electronic displays.

Example 2: vision correction display with dual mode of operation, suitable for normal vision and myopia

Example 1 is repeated, except that each array of OLED stripes 600 now has a pitch P of 315 $\mu$m. The perceived depth change F is again calculated:

$$F = f_L \left(1 + \frac{L}{L - P}\right)$$

$$\Leftrightarrow F = 500 \left(1 + \frac{300}{300 - 315}\right) \mu m = -9.5 \text{ cm}$$

Thus, by changing the pitch P of the OLED stripes, a perceived image pixel with a reduced depth of 9.5 cm, compared to the depth of the physical pixel, is now created.

By selecting different pitches, different perceived image depths are optained. For instance, for a pitch P of 305 $\mu$m rather than a pitch P of 315 $\mu$m between the OLED stripes, and a pitch L of 300 $\mu$m between the microlenses, the perceived image is at 29.5 cm in front of the display. This may be quite a lot for vision correction, but it may be useful for other applications.

Example 3: vision correction display with dual mode of operation, using a different way to select the mode

Reference is now made to FIG. 9. Example 1 is again repeated, but the transistor configuration is now such that a mode is selected by choosing an appropriate power input. The approach used in the example shown in FIG. 9 uses two power lines: a presbyopia mode powerline (P-$V_{DD}$) and a normal power line (N-$V_{DD}$). FIG. 9 illustrates that, by selecting the appropriate power line, either a normal or vision corrected pixel can be activated, and, by extension, the display 100 can be operated in either a normal or a vision corrected mode. Apart from this selection mechanism, the driving electronics can be the same as in traditional electronic displays.

Example 4: a specific predetermined pattern for the light sources

A specific way to organize the light sources 102, such as the OLED stripes 600 in example 1, in the vision correction display will be described from the viewpoint of a single physical pixel. Since the light sources within a physical pixel each correspond to a different array, this organization by extension also leads to an organization of the arrays.

Reference is made to FIG. 6 and FIG. 7. The OLED stripe top electrode 601 is 15 $\mu$m wide and there is a minimum separation distance between the top electrodes of 5 $\mu$m. Since the electroluminescent area 602 of an OLED stripe 600 was 5 $\mu$m, there is thus a minimum gap of 15 $\mu$m between the electroluminescent areas 602 of two adjacent OLED stripes 600. This gap limits the amount of OLED stripes 600 which can be fit into a single physical pixel and thus limits the amount of angles that can be used to construct the perceived image pixel 105. To overcome this, the second OLED stripe 600 is placed above or below the first one and shifted over the width of the electroluminescent area 602. This process is repeated for the third and fourth stripe, thereby bridging the distance between two neighbouring stripes 600. The fifth stripe 600 is then again displaced over the width of the electroluminescent area 602 but back at a similar height as the first stripe. This pattern is repeated for subsequent stripes. Since these OLED stripes 600 each correspond to a different array of OLED stripes and are thus all contacted by separate contact lines 201, it is important that the contact areas on these OLED stripes align with their corresponding contact lines 201. As such, in the present case, e.g. the fifth stripe is not located at exactly the same height as the first stripe, but slightly lower.

A pattern is thus created in which the contact lines corresponding to different arrays can run parallel to one another and contact their appropriate OLED stripes, corresponding to the appropriate emission angles, so that each array may create a single perceived image pixel at a certain depth.

Derived from this general pattern, and because the contact lines have a finite width, limits can be deduced for the angle the contact lines can make with respect to the length of a row of pixels (cf. supra).

It is to be understood that although preferred embodiments, specific constructions and configurations, as well as materials, have been discussed herein for devices according to the present invention, various changes or modifications in form and detail may be made without departing from the scope and spirit of this invention. For example, any formulas given above are merely representative of procedures that may be used. Functionality may be added or deleted from the block diagrams and operations may be interchanged among functional blocks. Steps may be added or deleted to methods described within the scope of the present invention.

**Claims**

1. A display (100) for generating an image with a perceived depth (104) of image pixels differing from the actual depth (103) of the image pixels (300), comprising:

   an array of microlenses (101) having a first pitch along a first direction (X);
   a plurality of arrays of light sources (102) underneath the array of microlenses (101), an array of light sources (102) having a second pitch along the first direction (X), the second pitch differing from the first pitch;
   means for addressing the plurality of arrays of light sources (102);
   wherein each array of light sources (102) corresponds to one perceived image pixel and wherein each light source (102) in an array is present underneath a different microlens (101);
   **characterized in that** the means for addressing the arrays of light sources (102) is adapted for addressing each light source (102) in one array of light sources (102) in unison.

2. The display (100) according to claim 1, wherein an array of light sources comprises three or more light sources (102), more preferably 20 or more, yet more preferably 50 or more, most preferably 90 or more.

3. The display (100) according to any of the previous claims, wherein a row of image pixels is formed by two or more arrays of light sources (102).

4. The display (100) according to any of the previous claims, wherein the means for addressing each light source (102) in one array of light sources (102) in unison comprises each light source (102) in the array being hardwired to a single driving transistor.

5. The display (100) according to claim 4, wherein the light sources (102) are arranged in a predetermined pattern and are electrically contacted by means of contact lines (201); the pattern being such that the contact lines (201) do not intersect.

6. The display (100) according to claim 5, wherein an array of light sources (102) comprises subgroups (202) each comprising a plurality of light sources (102); the subgroups (202) being shifted with respect to one another along the first direction (X) and the plurality of light sources (102) within a subgroup

(202) being shifted with respect to another light source (102) of the same subgroup both perpendicular to and along the first direction (X).

7. The display (100) according to claim 6, wherein contact lines (201) that connect two light sources (102) in a subgroup (202) of light sources (102) make an angle α with the first direction (X);
   **characterized in that** the angle α is smaller than $\arctan\left(\frac{H}{mP}\right)$, wherein H is the height of the row of image pixels (300), m is the number of light sources (102) in the subgroup (202) and P is the size of the second pitch.

8. The display (100) according to any of claims 6 or 7, wherein contact lines (201) that connect two light sources (102) in a subgroup (202) of light sources (102) make an angle α with the first direction;
   **characterized in that** the angle α is larger than $\arccos\left(\frac{H}{nT}\right)$, wherein H is the height of the row of image pixels, n is the number of contact lines (201) present in an image pixel and T is the minimum contact line pitch.

9. The display (100) according to any of the previous claims, wherein the second pitch is smaller than the first pitch.

10. The display (100) according to any of the previous claims, wherein the perceived depth (104) of the image pixels differs from the actual depth (103) of the image pixels by 10 to 200 cm, more preferably by 20 to 100 cm, yet more preferably by 25 to 50 cm.

11. The display (100) according to any of the previous claims, wherein the array of microlenses (101) comprises cylindrical microlenses oriented perpendicular to the first direction (X).

12. The display (100) according to any of the previous claims, wherein the array of light sources (102) comprises organic light emitting diodes.

13. The display (100) according to any of the previous claims, furthermore comprising one or more additional light sources (203) per pixel (300) such that in an alternative mode of operation an image may be created with a perceived depth (104) equal to the depth (103) of the source (102).

14. A method for generating an image with a perceived depth (104) of image pixels (300) differing from the actual depth (103) of the image pixels (300), comprising:

in an electronic device comprising a display, the display comprising an array of microlenses (101) having a first pitch along a first direction (X) and a plurality of arrays of light sources (102) underneath the array of microlenses (101), the arrays of light sources (102) having a second pitch along the first direction (X) differing from the first pitch,

addressing each light source (102) in one array of light sources (102) in unison.

15. A method for designing a display for generating an image with a perceived depth (104) of image pixels (300) differing from the actual depth (103) of the image pixels (300), comprising:

choosing a number x of pixels (300) per row in the display and a number *y* of rows, wherein the rows are oriented in a first direction (X);

choosing a number n of emission angles for constructing perceived pixels;

choosing a number m of rows of light sources (102) in each physical pixel;

providing an array of microlenses (101) with a first pitch in the first direction (X);

constructing a plurality of arrays of light sources (102) with a second pitch P in the first direction (X) differing from the first pitch, wherein each array comprises n light sources (102) and contact lines (201) contacting the *n* light sources (102), the *n* light sources (102) being addressable in unison and being ordered into subgroups of *m* light sources, the subgroups (202) shifted with respect to one another in the first direction (X), the *m* light sources (102) in a subgroup (202) shifted both in the first direction (X) and perpendicular to the first direction (X) such that a contact line (201) contacting the *m* light sources (102) makes an angle $\alpha$ with the first direction (X) with $\arccos\left(\frac{H}{nT}\right) < \alpha < \arctan\left(\frac{H}{mP}\right)$, arctan in which H is the height of a row of image pixels (300) and T is the minimum contact line pitch;

arranging the plurality of arrays of light sources (102), without overlapping them, such that only one light source (102) per array of light sources (102) is present beneath a microlens (101) and *n* arrays of light sources (102) are represented beneath a physical pixel (200).

FIG. 1

FIG. 2

**FIG. 3**

# FIG. 4

# FIG. 5

FIG. 6

FIG. 7

FIG. 8

FIG. 9

Europäisches
Patentamt

European
Patent Office

Office européen
des brevets

**EUROPEAN SEARCH REPORT**

Application Number

EP 15 20 2241

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 351 268 B1 (GENOE JAN [BE]) 26 February 2002 (2002-02-26) | 14 | INV. G02F1/1335 |
| A | * abstract; figures 3a-b, 4a-b, 20a-b, 22a-c, 23, 24a-b, 25, 27, 28 * * column 3, line 34 - column 14, line 45 * | 1-4, 9-11,15 | ADD. G02B27/00 |
| A | US 2011/285936 A1 (HORIKAWA YOSHIAKI [JP]) 24 November 2011 (2011-11-24) * paragraph [0047] - paragraph [0122]; figures 1-11 * | 1-15 | |
| A,D | VITOR F. PAMPLONA ET AL: "Tailored displays to compensate for visual aberrations", ACM TRANSACTIONS ON GRAPHICS (TOG), vol. 31, no. 4, 1 July 2012 (2012-07-01), pages 1-12, XP055257464, US ISSN: 0730-0301, DOI: 10.1145/2185520.2185577 * the whole document * | 1-15 | |

TECHNICAL FIELDS
SEARCHED      (IPC)

G02F
G02B
H04N

The present search report has been drawn up for all claims

| Place of search | Date of completion of the search | Examiner |
|---|---|---|
| Berlin | 19 May 2016 | A. Jacobs |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                         

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 15 20 2241

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2016

| Patent document cited in search report | | Publication date | Patent family member(s) | | Publication date |
|---|---|---|---|---|---|
| US 6351268 | B1 | 26-02-2002 | AU | 6500999 A | 17-04-2000 |
| | | | EP | 1119981 A1 | 01-08-2001 |
| | | | JP | 2002527000 A | 20-08-2002 |
| | | | US | 6351268 B1 | 26-02-2002 |
| | | | WO | 0019729 A1 | 06-04-2000 |
| US 2011285936 | A1 | 24-11-2011 | JP | 5420464 B2 | 19-02-2014 |
| | | | JP | 2011215550 A | 27-10-2011 |
| | | | US | 2011285936 A1 | 24-11-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **F.C. HUANG et al.** *ACM Trans. Graph.,* 2012, vol. 31.6, 185
- **V. PAMPLONA et al.** *ACM Trans. Graph.,* 2012, vol. 31.4, 81
- **F.C. HUANG et al.** *ACM Trans. Graph.,* 2014, vol. 33.4, 59